# EUROPEAN PATENT APPLICATION

(11) **EP 4 023 111 A1**
(43) Date of publication of application: **06.07.2022**
(21) Application number: 20859263.4
(22) Date of filing: 29.08.2020
(51) Int. Cl.: A47G 9/10, A61F 7/00, A61F 7/10

(54) **HEAD COOLING DEVICE AND HEAD COOLING METHOD**

(30) Priority: 30.08.2019 JP 2019158726
(71) Applicant: Konan Gakuen, Kobe-shi, Hyogo 658-0072 (JP); Iris Ohyama Inc., Sendai-shi, Miyagi 980-8510 (JP)
(72) Inventor: MAEDA, Kazuaki, Kobe-shi, Hyogo 658-0072 (JP); SUGIMORI, Toshiaki, Kakuda-shi, Miyagi 981-1596 (JP); SAKAMOTO, Susumu, Kakuda-shi, Miyagi 981-1596 (JP); SAITO, Yuta, Kakuda-shi, Miyagi 981-1596 (JP)
(74) Representative: Viering, Jentschura & Partner mbB Patent- und Rechtsanwälte
(86) International application number: PCT/JP2020/032791
(87) International publication number: WO 2021/040040

(57) **Abstract**

A head cooler improves sleep quality without overcooling the head of a sleeping person. A head cooler (1) includes a main body (3), a pillow (7), and a connector (5). The main body (3) sends out a liquid for circulation between the main body (3) and the pillow (7) through the connector (5). The main body (3) includes a temperature adjuster (31) that adjusts a temperature of the liquid, a pump (32) that sends out the liquid toward the pillow (7), and a controller (35) that controls the temperature adjuster (31) and the pump (32) to adjust a temperature of the pillow at a first target temperature of 22 to 24 °C until an elapsed time from start of cooling reaches a target time of 200 to 250 minutes.

## Description

### Technical Field

The present invention relates to, for example, a cooler for cooling the head of a resting person ready to sleep or a sleeping person.

### Background Art

A known cooler for cooling the head of a resting person ready to sleep or a sleeping person includes, for example, a head cooling apparatus 1 set and used in a bed at, for example, a hospital or a nursing home (refer to, for example, Patent Literature 1). As shown in FIG. 1, the head cooling apparatus 1 includes a head cooling pillow 2 (hereafter, pillow 2) as a liquid retainer on which the user's head is laid, and a mechanical unit 5 connected to the pillow 2 with flexible tubes 3 and 4. A cooling liquid circulates between the pillow 2 and the mechanical unit 5.

### Citation List

### Patent Literature

Patent Literature 1: Japanese Unexamined Patent Application Publication No. 2013-126523, paragraph 0027

### Summary of Invention

### Technical Problems

The above apparatus can overcool the head of a sleeping person. A person who has the head continuously cooled while sleeping may complain about overcooling sensation. Overcooling can also induce prolonged muscle tone in the neck and the upper body and induce blood circulation failure.

These can degrade sleep quality.

One or more aspects of the present invention are directed to a head cooler and a head cooling method for improving sleep quality without overcooling the head of a sleeping person.

### Solution to Problems

A head cooler according to an aspect of the present invention includes a main body, a pillow, and a connector. The main body sends out a liquid for circulation between the main body and the pillow through the connector. The main body includes a temperature adjuster that adjusts a temperature of the liquid, a pump that sends out the liquid toward the pillow, and a controller that controls the temperature adjuster and the pump to adjust a temperature of the pillow at a first target temperature of 22 to 24 °C until an elapsed time from start of cooling reaches a target time of 200 to 250 minutes.

A head cooling method according to an aspect of the present invention is a method for cooling a head of a resting person ready to sleep or a sleeping person. The method includes circulating a liquid sent out from a main body between the main body and a pillow through a connector, and adjusting a temperature of the pillow at 22 to 24 °C until an elapsed time from start of cooling reaches a target time of 200 to 250 minutes.

### Advantageous Effects of Invention

The above structure can improve sleep quality without overcooling the head of a sleeping person.

### Brief Description of Drawings

FIG. 1 is a schematic diagram of a head cooler according to a first embodiment.
FIG. 2 is a flowchart of processing performed by a controller.
FIG. 3 is a graph illustrating a human body temperature change (biological rhythms) during sleep.
FIG. 4 is a flowchart of a part of processing in a second embodiment.

### Description of Embodiments

### Overview

A head cooler according to one embodiment includes a controller that controls a temperature adjuster and a pump to adjust the temperature of a pillow at a first target temperature of 22 to 24 degrees Celsius (°C) until an elapsed time from the start of cooling reaches a target time of 200 to 250 minutes. The circulation time is 200 to 250 minutes, and thus prolonged cooling of the head can be avoided. The temperature of the pillow is adjusted at 22 to 24 °C. A reference temperature is 24 °C. A person who intends a cooler environment can select 22 °C around which the person can obtain subjective feeling of sleep without any substantive difference in sleep quality. The temperature of the pillow herein refers to the temperature of the surface of the pillow (the surface in contact with the head) in Celsius.

The temperature of the pillow adjusted at 22 to 24 °C can shorten sleep latency and rapidly induce deep sleep. The temperature of the pillow is adjusted at 22 to 24 °C until 200 to 250 minutes elapses after the start of cooling. This improves sleep efficiency.

The sleep efficiency herein refers to the value (%) obtained by dividing the sleep time (total sleep time) excluding night awakenings during sleep by the time from sleep onset to sleep offset (total time in bed). The sleep latency and the sleep efficiency are measured using an actigraph and an electroencephalograph.

In the head cooler according to another embodiment, the controller adjusts the temperature of the pillow at a second target temperature higher than the first target temperature after elapse of the target time. This structure improves sleep quality.

Sleep quality herein includes three factors, the shortness of sleep latency, sleep efficiency, and the absence of discomfort upon waking.

In the head cooler according to another embodiment, the controller receives an input of a bedtime to control the pillow to be at the first target temperature at the bedtime. This structure allows cooling of the head of the resting person ready to sleep at the first target temperature.

A head cooling method according to one embodiment includes adjusting the temperature of the pillow at 22 to 24 °C until an elapsed time from start of cooling reaches a target time of 200 to 250 minutes. The circulation time is 200 to 250 minutes, and thus prolonged cooling of the head can be avoided. The temperature of the pillow adjusted at 22 to 24 °C can shorten sleep latency and rapidly induce deep sleep. The temperature of the pillow is adjusted at 22 to 24 °C until 200 to 250 minutes elapses after the start of cooling. This improves sleep efficiency.

The head cooling method may include adjusting the temperature of the pillow at the second target temperature higher than the first target temperature after elapse of the target time or may include receiving an input of a bedtime to adjust the temperature of the pillow at the first target temperature at the bedtime.

### First Embodiment

### 1. Head Cooler

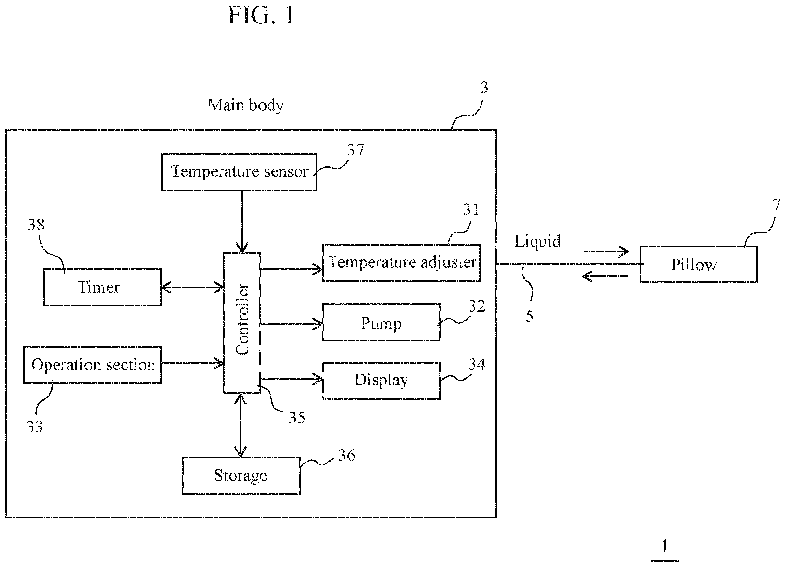

As shown in FIG. 1, a head cooler 1 includes a main body 3, a connector 5, and a pillow 7. The head cooler 1 cools the head of a resting person ready to sleep or a sleeping person by circulating a liquid at a temperature adjusted in the main body 3 between the main body 3 and the pillow 7 through the connector 5.

### (1) Main Body

As shown in FIG. 1, the main body 3 includes a temperature adjuster 31, a pump 32, an operation section 33, a display 34, a controller 35, a storage 36, a temperature sensor 37, and a timer 38. The main body 3 may contain the temperature adjuster 31 and other components in a single housing or in multiple housings. For example, the main body 3 may include the temperature adjuster 31 and the pump 32 in different housings.

The temperature adjuster 31 adjusts the liquid to be sent out of and received into the pump 32 at a constant temperature or a temperature within a predetermined range. The liquid sent out of the main body 3 is heated in the pillow 7 by the head of the resting person ready to sleep or the sleeping person. The temperature adjuster 31 thus mostly cools the liquid. The temperature adjuster 31 includes, for example, a Peltier element and cools the liquid through voltage application.

The pump 32 receives the liquid for circulation from the pillow 7 and sends out the received liquid back to the pillow 7. The pump 32 is connected to the connector 5.

The operation section 33 includes, for example, a power switch for turning on and off the cooler, a start switch for starting a mode (hereafter, a cooling mode) for cooling the head of the resting person ready to sleep or the sleeping person, and a stop switch for stopping the cooling mode.

The display 34 displays, for example, the elapsed time from the start of the cooling mode and the temperature of the pillow 7 (or the liquid).

The controller 35 includes, for example, a central processing unit (CPU) to control the head cooler 1 (main body 3) entirely based on a computer program and various items of setting data stored in the storage 36.

In response to an operation on the start switch for the cooling mode, the controller 101 controls the temperature adjuster 31 using the temperature detected by the temperature sensor 37 and controls the temperature adjuster 31 and the pump 32 using the time measured by the timer 38. More specifically, in response to the start of the cooling mode, the controller 101 controls the temperature adjuster 31 and the pump to adjust the temperature of the liquid (water) flowing through the pillow 7 to remain at 22 to 24 °C (e.g., 24 °C) for 200 to 250 minutes.

The controller 35 performs processing corresponding to an operation received from the operator through the operation section 133 and causes the display 34 to display the elapsed time during the cooling mode.

The storage 102 specifically includes a random-access memory (RAM), a read-only memory (ROM), or a flash memory for the work of the controller 35. The storage 102 stores a computer program for implementing the head cooling method according to the present embodiment as well as various items of setting data. The items of setting data include, for example, the temperature of the pillow 7 in the cooling mode and the duration of the cooling mode.

The temperature sensor 37 detects the temperature of the pillow 7 or the temperature corresponding to the temperature of the pillow 7 (the temperature of the liquid or the temperature corresponding to the temperature of the liquid). The timer 38 starts measurement and outputs the elapsed time to the controller 35 in response to an instruction from the controller 35.

### (2) Connector and Pillow

The connector 5 includes a tube and connector ends at both ends of the tube. The connector 5 includes an upstream channel from the main body 3 to the pillow 7 and a downstream channel from the pillow 7 to the main body 3. The upstream end of the upstream channel and the downstream end of the downstream channel are connected to the main body 3, and the downstream end of the upstream channel and the upstream end of the downstream channel are connected to the pillow 7.

The pillow 7 includes an internal channel. The internal channel has an inlet at an upstream end of the internal channel connected to the downstream end of the upstream channel of the connector 5 and an outlet at a downstream end of the internal channel connected to the upstream end of the downstream channel of the connector 5.

### 2. Controlling Cooling Mode

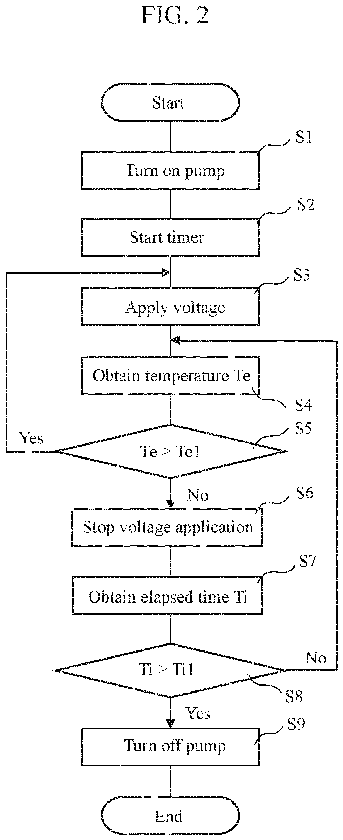

The cooling mode is controlled by the controller 35 as described below with reference to FIG. 2.

In response to an operation on the start switch in the operation section 33 performed by an operator, the controller 35 reads a program for the cooling mode from the storage 36 and executes the program. The controller 35 turns on the pump (S1), starts the timer 38 (S2), and applies voltage to the Peltier element (S3).

This starts circulation of the liquid between the main body 3 and the pillow 7.

The controller 35 obtains a temperature Te measured by the temperature sensor 37 (S4). In response to the temperature Te being higher than a target temperature (first target temperature) Te1 (Yes in step S5), the controller 35 returns to step S3 and repeats the processing in steps S3 to S5 until the temperature Te measured by the temperature sensor 37 becomes the target temperature Te1 or lower. The target temperature Te1 is stored in the storage 36. The target temperature Te1 is, for example, 24 °C.

In response to the temperature Te being lower than or equal to the target temperature Te1 (No in step S5), the controller 35 stops applying voltage to the Peltier element (S6) and obtains an elapsed time Ti measured by the timer 38 (S7). In response to the elapsed time Ti not reaching a target time (first target time) Ti1 (No in step S8), the controller 35 returns to step S4. In response to the elapsed time Ti reaching the target time Ti1 (Yes in step S8), the controller 35 turns off the pump (S9) and ends the processing. The target time Ti1 is stored in the storage 36. The target time Ti1 is, for example, 240 minutes (4 hours).

### 3. Sleep Efficiency

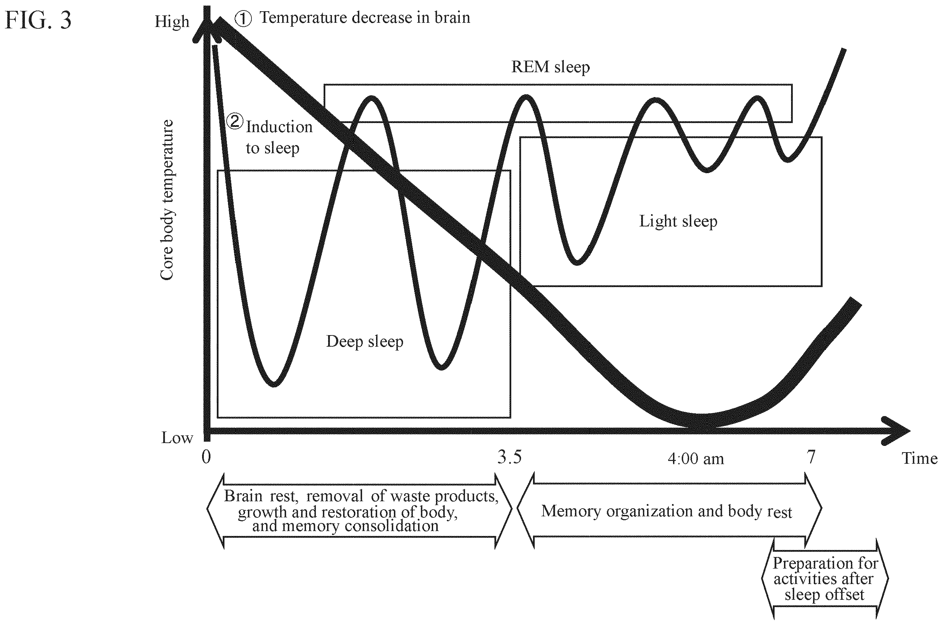

FIG. 3 is a graph illustrating the depth of sleep and the core body temperature over time after sleep onset.

For ease of explanation, FIG. 3 collectively shows examples of the depth of sleep and the core body temperature (sleep rhythm) that vary among different people, genders, and ages.

### (1) Depth of Sleep

Night sleep typically includes several repeats of a pair of non-rapid eye movement (non-REM) sleep and REM sleep after sleep onset and before sleep offset. The pair of non-REM sleep and REM sleep is referred to as a sleep cycle that occurs periodically every 90 to 120 minutes.

Up to about 3.5 hours from sleep onset, deep sleep occurs, and non-REM sleep with a greater depth (the lower part in the graph indicated by the vertical axis on the left) and REM sleep are repeated twice. The deep sleep is followed by, before sleep offset, relatively light sleep in which REM sleep and non-REM sleep with a moderate depth (the middle part in the graph indicated by the vertical axis on the left) or non-REM sleep with a less depth (the upper part in the graph indicated by the vertical axis on the left) occur repeatedly. This cycle is referred to as sleep rhythm.

In about 3.5 hours from sleep onset, the brain rests, waste products are removed, the body grows and recovers, and memory is consolidated, for example. In a period from about 3.5 hours after sleep onset to sleep offset, the brain rests, memory is organized, and the body rests, for example. The period from when the core body temperature is the lowest to before sleep offset is used, for example, to prepare for activities after the sleep offset.

### (2) Core Body Temperature

The skin temperature (body surface temperature) starts increasing about one hour before habitual bedtime. The core body temperature decreases as the skin temperature increases.

The core body temperature decreases until about 5.5 hours after sleep onset and then increases toward habitual waking time. For humans, the core body temperature typically is the lowest at 4:00 to 5:00 am, although the time varies among different genders and ages.

The core body temperature decreases efficiently when the brain or head is cooled.

### (3) Depth of Sleep and Core Body Temperature

As shown in FIG. 3, after sleep onset, the core body temperature decreases and the depth of sleep becomes deeper. More specifically, rapidly lowering the core body temperature after the sleep onset induces deep sleep.

The sleep enters a lighter phase from the end of the decrease in the core body temperature after sleep onset (about 4.5 hours after the sleep onset). The core body temperature then increases toward the sleep offset. More specifically, the core body temperature increasing without being restricted allows comfortable waking.

### (4) Head Cooler

The head cooler 1 according to one or more embodiments of the present invention is used to cool a head to allow optimal sleep based on the above relationship between the depth of sleep and the core body temperature.

The head cooler 1 circulates the liquid to maintain the temperature of the pillow 7 at 22 to 24 °C. This allows the resting person ready to sleep to fall asleep comfortably without feeling too cold on the pillow 7.

The inventors have noticed that the pillow 7 at 22 to 24 °C allows the sleeping person to have deeper sleep than at other temperatures for cooling.

The head cooler has no control over, for example, the bedtime or the sleep onset latency of the sleeping person, and takes about 30 minutes to cool the pillow to the target temperature Te1. For smooth induction to sleep, the pillow 7 may be cooled by the bedtime.

### Second Embodiment

In the first embodiment, the cooling mode is controlled to stop circulation of the liquid in response to the elapsed time Ti from the operation on the start switch reaching the first target time Ti 1. In some embodiments, after the first target time Ti 1 elapses, the liquid may be circulated at a second target temperature Te2 adjusted to be higher than the first target temperature Te1 used for cooling during the first target time Ti1, or the liquid may be circulated under no voltage.

The second target temperature Te2 is lower than the temperature of the pillow raised after the cooling and the circulation of the liquid are stopped in response to the elapse of the first target time Ti1. This reduces the likelihood of the temperature of the pillow increasing excessively.

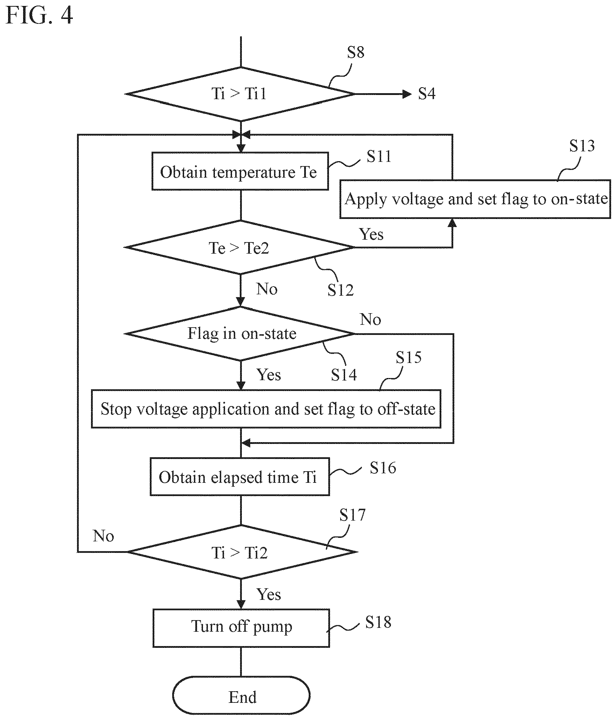

The pillow 7 is controlled to be at the second target temperature Te2 by a controller until the end of the cooling mode (the time elapsed before the end of the cooling mode is referred to as a second target time Ti2) as described below with reference FIG. 4.

A controller 135 according to a second embodiment (although not shown, the controller 135 is distinguished from the controller 35 in the first embodiment) performs the processing according to the second embodiment after the processing in steps S1 to S8 (refer to FIG. 2) described in the first embodiment. FIG. 4 shows step S8 and subsequent steps (described below).

In response to the elapsed time Ti reaching the first target time Ti1 (Yes in step 8), the controller 135 obtains the temperature Te measured by the temperature sensor 37 (S11). In response to the temperature Te being higher than the second target temperature Te2 (Yes in step S12), the controller 135 applies voltage to the Peltier element and sets a flag to an on-state (S13) to indicate that voltage is being applied. The controller 135 then returns to step S11. The second target temperature Te2 is stored in the storage 36. The second target temperature Te2 is, for example, 28 °C.

In response to the temperature Te being less than or equal to the second target temperature Te2 (No in step S12) and the flag being in the on-state (Yes in step S14) indicating that voltage is being applied, the controller 135 stops applying voltage and sets the flag to an off-state (indicating that voltage is not being applied) (S15). The controller 135 then advances to step S16. In response to the flag being in the off-state (No in step 14), the controller advances to step S16.

In step S16, the controller 135 obtains the elapsed time Ti measured by the timer 38. In response to the elapsed time Ti not reaching the second target time Ti2 (No in step S17), the controller 135 returns to step S11. In response to the elapsed time Ti reaching the second target time Ti2 (Yes in step S17), the controller 135 turns off the pump (S18) and ends the processing. The second target time Ti2 is stored in the storage 36. The second target time Ti2 is, for example, 480 minutes (8 hours).

The control described above reduces the likelihood of the core body temperature increasing excessively before sleep offset. This improves sleep quality and allows comfortable waking.

The pillow 7 is cooled for 240 minutes after the start of cooling, and is then maintained at 28 °C. This reduces the likelihood of the sleeping person feeling hot and waking up and improves sleep quality and allows comfortable waking.

Continuously cooling the head until morning (a whole day and night) may reportedly reduce the feeling of being well-rested upon sleep offset.

The head cooler and the head cooling method according to the above embodiments are not limiting and may be modified as described below. The embodiments and modifications may be combined with one another, or modifications may be combined with one another.

Examples that are not described in the embodiments or modifications or design changes without departing from the gist of the invention also fall within the scope of the present invention.

(1) In the first and second embodiments, the cooling mode is controlled to stop cooling upon the elapsed time Ti from the operation on the start switch reaching 240 minutes. More specifically, the elapsed time Ti is measured from when the start switch is operated, including the cooling time (e.g., 20 to 40 minutes) to cool the pillow 7 to 22 to 24 °C.

In some embodiments, the elapsed time Ti may by measured from when the temperature of the pillow 7 becomes 22 to 24 °C (e.g., when the processing is first reaches step S6 in FIG. 2). In this case, the elapsed time Ti is 200 to 220 minutes (e.g., 210 minutes).

More specifically, the controller controls the temperature adjuster and the pump to maintain the pillow at 22 to 24 °C for 200 to 220 minutes.

(2) The temperature sensor 37 measures the temperature of the part defining the channel for the liquid. The controller prestores the temperature of the part at which the temperature of the pillow becomes 22 to 24 °C and uses the temperature of the part to perform control.

In some embodiments, the temperature of the liquid may be directly measured by a temperature sensor. In this case as well, the surface temperature of the pillow is associated with the liquid temperature. The temperature sensor may be installed on the pillow and may wirelessly transmit measurement results to the controller, for example.

(3) In the first and second embodiments, the cooling mode is started in response to an operator operating the start switch in the operation section 33. In some embodiments, for example, the bedtime of a sleeping person may be input. The cooling of the liquid may then be started to achieve the first target temperature at the bedtime, or more specifically, before the person goes to bed.

### Reference Signs List

- 1: head cooler
- 3: main body
- 5: connector
- 7: pillow
- 31: temperature adjuster
- 33: pump
- 35: controller

## Claims

1. A head cooler, comprising:
a main body;
a pillow; and
a connector,
wherein the main body sends out a liquid for circulation between the main body and the pillow through the connector,
the main body includes
a temperature adjuster configured to adjust a temperature of the liquid,
a pump configured to send out the liquid toward the pillow, and
a controller configured to control the temperature adjuster and the pump to adjust a temperature of the pillow at a first target temperature of 22 to 24 °C until an elapsed time from start of cooling reaches a target time of 200 to 250 minutes.

2. The head cooler according to claim 1, wherein
the controller adjusts the temperature of the pillow at a second target temperature higher than the first target temperature after elapse of the target time.

3. The head cooler according to claim 1 or claim 2, wherein
the controller receives an input of a bedtime to control the pillow to be at the first target temperature at the bedtime.

4. A head cooling method for cooling a head of a resting person ready to sleep or a sleeping person, the method comprising:
circulating a liquid sent out from a main body between the main body and a pillow through a connector; and
adjusting a temperature of the pillow at 22 to 24 °C until an elapsed time from start of cooling reaches a target time of 200 to 250 minutes.
